(19) Europäisches Patentamt
European Patent Office
Office européen des brevets

(11) EP 3 805 195 B1

(12) **EUROPEAN PATENT SPECIFICATION**

(45) Date of publication and mention of the grant of the patent:
**22.02.2023 Bulletin 2023/08**

(21) Application number: **19812018.0**

(22) Date of filing: **28.05.2019**

(51) International Patent Classification (IPC):
***C07C 59/66*** *(2006.01)* ***C07C 51/43*** *(2006.01)*

(52) Cooperative Patent Classification (CPC):
(C-Sets available)
**C07C 59/66; C07C 51/43;** C07B 2200/13 (Cont.)

(86) International application number:
**PCT/JP2019/021005**

(87) International publication number:
**WO 2019/230685 (05.12.2019 Gazette 2019/49)**

(54) **CRYSTAL OF 2,2'-BIS(CARBOXYMETHOXY)-1,1'-BINAPHTHYL**

KRISTALL VON 2,2'-BIS(CARBOXYMETHOXY)-1,1'-BINAPHTHYL

CRISTAL DE 2,2'-BIS(CARBOXYMÉTHOXY)-1,1'-BINAPHTYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2018 JP 2018104335**
**26.04.2019 JP 2019085666**

(43) Date of publication of application:
**14.04.2021 Bulletin 2021/15**

(73) Proprietor: **Honshu Chemical Industry Co., Ltd.**
**Tokyo 103-0027 (JP)**

(72) Inventors:
- **SAKUMA Daichi**
  **Wakayama-shi, Wakayama 641-0007 (JP)**
- **YAMANE Kentaro**
  **Wakayama-shi, Wakayama 641-0007 (JP)**
- **MIZOGUCHI Shun**
  **Wakayama-shi, Wakayama 641-0007 (JP)**
- **SUTO Takeru**
  **Wakayama-shi, Wakayama 641-0007 (JP)**
- **KODERA Masato**
  **Wakayama-shi, Wakayama 641-0007 (JP)**

(74) Representative: **Grünecker Patent- und Rechtsanwälte**
**PartG mbB**
**Leopoldstraße 4**
**80802 München (DE)**

(56) References cited:
**WO-A1-2019/043060 JP-A- 2001 072 872**

- **JI, FENGYING et al.: "Snthesis of 1,1'-bi-2,2'-naphthol diethers", Zhongshan Daxue Xuebao, Ziran Kexueban, vol. 35, no. 3, May 1996 (1996-05), pages 120-123, XP055756968,**
- **GHOSH, KUMARESH et al.: "rac)-1,1'-Binaphthyl-Based Simple Receptors Designed for Fluorometric Discrimination of Maleic and Fumaric Acids", The Journal of Physical Chemistry B, vol. 115, no. 26, 2011, pages 8597-8608, XP055756971,**
- **LEHN, JEAN MARIE et al.: "Synthesis and properties of chiral macrotricyclic ligands. Complexation and transport of chiral molecular cations and anions", Helvetica Chimica Acta, vol. 61, no. 7, 1978, pages 2407-18, XP055756973,**
- **DIETRICH, BERNARD et al.: "Chiral macrobicyclic and macrotricyclic ligands", Angewandte Chemie, vol. 86, no. 12, 1974, pages 443-444, XP055756975,**
- **COLUCCINI, CARMINE et al.: "Locked chromophores as CD and NMR probes for the helical conformation of tetraamidic macrocycles", Organic & Biomolecular Chemistry, vol. 8, no. 8, 2010, pages 1807-1815, XP055756979,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BICKLEY, JAMIE et al.: "Dirhodium(II) carboxylate complexes as building blocks. cis-Chelating dicarboxylic acids designed to bridge the dinuclear core", New Journal of Chemistry, vol. 28, no. 3, 2004, pages 425-433, XP055756981,**


(52) Cooperative Patent Classification (CPC): (Cont.)

C-Sets
**C07C 51/43, C07C 59/66**

**Description**

TECHNICAL FIELD

The present invention relates to a novel crystal of a dicarboxylic acid compound and a method for producing the crystal. More specifically, the present invention relates to a crystal of 2,2'-bis(carboxymethoxy)-1,1'-binaphthyl, which has an endothermic peak top temperature falling within a specific range as measured by differential scanning calorimetry and also has an aerated bulk density falling within a specific range.

BACKGROUND ART

In recent years, polyester resins and polyester carbonate resins each produced using a dicarboxylic acid component having a binaphthalene backbone as a polymerization component have been expected as raw materials for optical members such as an optical disk, a transparent electrically conductive substrate and an optical filter, because these resins have excellent optical characteristics including high refractive indexes and low birefringence and also have high levels of heat resistance. Among these resins, particularly a resin produced using 2,2'-bis(carboxymethoxy)-1,1'-binaphthyl (also referred to as "compound A", hereinafter) which has a chemical structure represented by the chemical formula shown below as a polymerization component has been focused due to the excellent optical characteristics thereof (see, for example, Patent Literatures 1 to 4).

[Chemical formula 1]

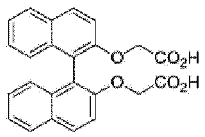

As the method for producing compound A represented by the above-shown formula, a method is known, in which 1,1'-binaphthalene-2,2'-diol is reacted with a halogenated acetate ester such as ethyl chloroacetate to produce a diester and the diester is hydrolyzed as illustrated in the reaction formula shown below (see, for example, Patent Literature 5). However, it is often the case that compound A produced by this reaction is not to be purified and a crude product thereof is converted to an acid chloride with thionyl chloride or oxalyl chloride upon use. Therefore, the purification method for compound A has not been studied or reported yet.

[Chemical formula 2]

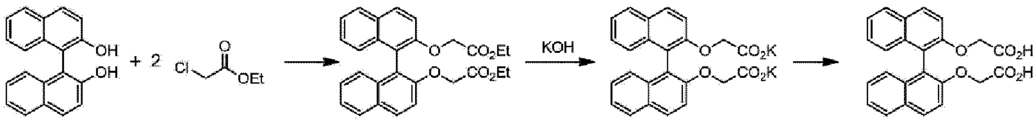


The present inventors have repeatedly produced compound A, and it is found that the dicarboxylic acid compound has several crystal forms having different properties from each other. The Journal of Physical Chemistry B, vol. 115, no. 26, 2011, pages 8597-8608 discloses 2,2'-bis(carboxymethoxy)-1,1'-binaphthyl in white solid form.

CITATION LIST

PATENT LITERATURE

Patent Literature 1: Japanese Patent Publication Laid-open No. 2001-072872
Patent Literature 2: Japanese Patent Publication Laid-open No. 2018-002893
Patent Literature 3: Japanese Patent Publication Laid-open No. 2018-002894
Patent Literature 4: Japanese Patent Publication Laid-open No. 2018-002895
Patent Literature 5: Japanese Patent Publication Laid-open No. 2008-024650

## SUMMARY OF THE INVENTION

### PROBLEM TO BE SOLVED BY THE INVENTION

In light of the aforementioned situations, the present invention has been made. The present invention addresses the problem of providing a novel crystal of compound A which is suitable as a resin raw material having excellent optical characteristics.

### MEANS FOR SOLVING THE PROBLEM

The present inventors have made extensive and intensive studies for solving the above-mentioned problem, and have found that a crystal of compound A which has an endothermic peak top temperature falling within a specific range as determined by differential scanning calorimetry and also has an aerated bulk density falling within a specific range can be produced by the crystallization with a specific solvent. This finding leads to the accomplishment of the present invention.

The present invention is as follows.

1. A crystal of 2,2'-bis(carboxymethoxy)-1,1'-binaphthyl, the crystal having an endothermic peak top temperature within a range of 215 to 220°C as measured by differential scanning calorimetry and also having an aerated bulk density within a range of 0.3 to 0.6 g/cm$^3$.
2. The crystal of 2,2'-bis(carboxymethoxy)-1,1'-binaphthyl according to item 1, wherein Hazen color number (APHA) of a 30% by weight solution prepared by dissolving the crystal in tetrahydrofuran is 100 or less.
3. A method for producing the crystal according to item 1 or 2, the method including carrying out crystallization with any one solvent selected from the following solvents (1) to (5):

   (1) at least one solvent selected from linear ketone solvents each having 5 to 8 carbon atoms in total;
   (2) at least one solvent selected from cyclic ketone solvents each having 5 to 8 carbon atoms in total;
   (3) at least one solvent selected from cyclic ether solvents each having 4 to 8 carbon atoms in total;
   (4) at least one solvent selected from cyclic ester solvents each having 4 to 8 carbon atoms in total; and
   (5) a mixed solvent of at least one solvent selected from linear ketone solvents each having 3 to 8 carbon atoms in total and water.

4. The method for producing according to item 3, wherein the method includes carrying out crystallization with any one solvent selected from the following solvents (1') to (5'):

   (1') methyl isobutyl ketone or 2-octanone;
   (2') cyclohexanone;
   (3') tetrahydrofuran or 1,4-dioxane;
   (4') γ-butyrolactone or γ-valerolactone; and
   (5') a mixed solvent of acetone and water or a mixed solvent of methyl ethyl ketone and water.

### ADVANTAGEOUS EFFECTS OF INVENTION

According to the present invention, it is possible to provide a crystal of compound A which has an endothermic peak top temperature falling within a specific range as measured by differential scanning calorimetry and also has an aerated bulk density falling within a specific range.

The crystal of the present invention has such characteristic properties that the endothermic peak top temperature as measured by differential scanning calorimetry is high and the aerated bulk density is also high. Therefore, it is possible to produce, use, transport or the like the compound with high efficiency. Furthermore, it is possible to prevent the flying of dusts during the production of an optical resin using the crystal of the compound as a raw material, and therefore the adhesion of the dusts onto a production facility or the clogging in the production facility with the dusts can be prevented. In addition, the capacity of a reaction vessel can be reduced when the crystal is used as a reaction raw material. As a result, the improvement in production efficiency can be expected. It is also possible to reduce the capacity of a container to be used for the transportation of the crystal. As a result, excellent effects with respect to operability, such as the reduction in transportation cost, can be achieved.

In addition, the crystal of the present invention is produced by a crystallization operation, and therefore can have excellent characteristic properties such as high purity and reduced coloring.

Namely, the crystal and the method for producing the crystal according to the present invention are extremely

useful in industrial applications including the use as a resin raw material.

BRIEF DESCRIPTION OF THE DRAWINGS

Fig. 1 is a chart diagram showing the data of the differential scanning calorimetry of a crystal obtained in Example 1.
Fig. 2 is a chart diagram showing the data of the differential scanning calorimetry of a crystal obtained in Example 2.
Fig. 3 is a chart diagram showing the data of the differential scanning calorimetry of a crystal obtained in Example 3.
Fig. 4 is a chart diagram showing the data of the differential scanning calorimetry of a solid material obtained in Comparative Example 1.
Fig. 5 is a chart diagram showing the data of the differential scanning calorimetry of a solid material obtained in Comparative Example 2.

DESCRIPTION OF EMBODIMENTS

Hereinafter, the present invention will be described in detail.
Compound A of the present invention is a compound represented by the following chemical formula.

[Chemical formula 3]

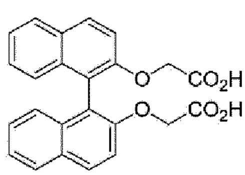

<Synthesis method>

The method for synthesizing compound A of the present invention is not particularly limited. One example of the method is a production method in which 1,1'-binaphthalene-2,2'-diol that is a known compound is reacted with a halogenated acetate ester such as ethyl chloroacetate to produce a diester and then the diester is hydrolyzed.

[Chemical formula 4]

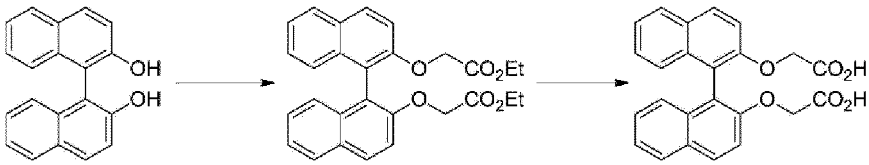

<Crystallization step>

The production method of the present invention is characterized by including carrying out crystallization with any one solvent selected from the following solvents (1) to (5):

(1) at least one solvent selected from linear ketone solvents each having 5 to 8 carbon atoms in total;
(2) at least one solvent selected from cyclic ketone solvents each having 5 to 8 carbon atoms in total;
(3) at least one solvent selected from cyclic ether solvents each having 4 to 8 carbon atoms in total;
(4) at least one solvent selected from cyclic ester solvents each having 4 to 8 carbon atoms in total; and
(5) a mixed solvent of at least one solvent selected from linear ketone solvents each having 3 to 8 carbon atoms in total and water.

Examples of the linear ketone solvent having 5 to 8 carbon atoms in total (1) which can be used in the present invention include diethyl ketone, methyl isobutyl ketone, methyl amyl ketone and 2-octanone. Among these solvents, methyl isobutyl ketone, methyl amyl ketone and 2-octanone each having low solubility in water are preferred. Examples

of the cyclic ketone solvent having 5 to 8 carbon atoms in total (2) which can be used in the present invention include cyclopentanone, cyclohexanone, cycloheptanone and cyclooctanone. Among these solvents, cyclopentanone and cyclohexanone are preferred. Examples of the cyclic ether solvent having 4 to 8 carbon atoms in total (3) which can be used in the present invention include oxetane, tetrahydrofuran, tetrahydropyran and 1,4-dioxane. Among these solvents, tetrahydrofuran and 1,4-dioxane are preferred. Examples of the cyclic ester solvent having 4 to 8 carbon atoms in total (4) which can be used in the present invention include γ-butyrolactone, γ-valerolactone, σ-valerolactone and ε-caprolactone. Among these solvents, γ-butyrolactone and γ-valerolactone are preferred. Examples of the linear ketone (5) which is to be used in a mixed solvent with water include acetone, methyl ethyl ketone, diethyl ketone, methyl isobutyl ketone, methyl amyl ketone and 2-octanone. Among these solvents, acetone and methyl ethyl ketone each having high solubility in water are preferred. Water which can be used in the present invention is not particularly limited. For example, tap water, distilled water, ion exchange water and natural water can be used as required.

Examples of the form of compound A to be used in the crystallization step of the present invention include: a crude crystal produced by treating a reaction solution containing compound A; a crystal produced by recrystallizing the crude crystal; and a residual liquid produced by removing a solvent from a solution containing compound A by distillation. Alternatively, the form of compound A may be an amorphous form. Compound A has two types of enantiomers. In the present invention, compound A to be used in the crystallization step is preferably of a racemic form, and the crystal produced by the crystallization step is also preferably of a racemic form.

In the case where the linear ketone solvent (1) (e.g., methyl isobutyl ketone and methyl amyl ketone) is used as is (i.e., not in the form of a mixed solvent with water), the amount of the solvent to be used for the dissolution of compound A therein is preferably 250 to 1000 parts by weight, more preferably 300 to 800 parts by weight, still more preferably 400 to 600 parts by weight, relative to 100 parts by weight of compound A contained in the crystals or solution to be used. In the case where it is intended to dissolve all of the crystals by increasing the temperature, the dissolution may be carried out under ambient pressure or under increased pressure. When the amount of the linear ketone solvent (1) used is 450 parts by weight or less, it is preferred to dissolve the crystals under increased pressure. It is possible to crystallize compound A by directly cooling a solution produced by dissolving compound A in the ketone solvent. Alternatively, it is possible to precipitate crystals while distilling the ketone solvent away from the solution, or it is possible to precipitate crystals by cooling a solution produced after the distillation of the ketone solvent away from the solution. In the case where compound A is dissolved in a linear ketone solvent in an amount of 500 parts by weight or more relative to 100 parts by weight of compound A, it is preferred to distil the ketone solvent away from the solution in order to improve the yield of the crystals. With respect to the amount of the linear ketone solvent in a residual liquid obtained after the distillation away of the solvent, it is preferred to adjust the amount to 150 to 450 parts by weight, more preferably 200 to 400 parts by weight, still more preferably 250 to 300 parts by weight, relative to 100 parts by weight of the crystals used. The temperature to be employed for the precipitation of the crystals is preferably 90 to 130°C, more preferably 95 to 105°C. The period of time to be employed for the distillation away of the solvent is preferably 2 to 15 hours, more preferably 4 to 10 hours, still more preferably 6 to 8 hours. Alternatively, it is also possible to add the linear ketone solvent to an aqueous solution having an alkali metal salt of compound A dissolved therein and then further add an acid to the resultant solution to prepare a ketone solution of compound A. Subsequently, an aqueous layer is separated off to obtain a solvent layer, then a water washing procedure comprising adding water to the obtained solvent layer and stirring the resultant solution to separate off an aqueous layer is carried out, and then the crystals of compound A can be precipitated by carrying out the same procedure as mentioned above.

In the case where any one of the cyclic ketone solvent (2), the cyclic ether solvent (3) or the cyclic ester solvent (4) is used, the amount of the solvent to be used for the dissolution of compound A therein is preferably 50 to 600 parts by weight, more preferably 50 to 400 parts by weight, still more preferably 100 to 200 parts by weight, relative to 100 parts by weight of compound A contained in crystals or a solution to be used. When all of the crystals are dissolved by increasing the temperature, the dissolution may be carried out under ambient pressure or under increased pressure. It is possible to crystallize compound A by directly cooling a solution produced by dissolving compound A in any one of the cyclic ketone solvent (2), the cyclic ether solvent (3) or the cyclic ester solvent (4). Alternatively, it is possible to precipitate crystals while distilling the solvent away from the solution, or it is possible to precipitate crystals by cooling a solution produced after the distillation of the solvent away from the solution. In the case where the cyclic ketone solvent is used, the temperature to be employed for the precipitation of the crystals is preferably 90 to 110°C, more preferably about 100°C. In the case where the cyclic ether solvent is used, the temperature is preferably 55 to 75°C, more preferably 60 to 70°C. In the case where the cyclic ester solvent is used, the temperature is preferably 90 to 130°C, more preferably 115 to 125°C.

In the case where the mixed solvent (5) composed of the linear ketone solvent and water is used, the concentration of the linear ketone solvent in the mixed solvent to be used for the dissolution of compound A is preferably 95 to 65% by weight, more preferably 90 to 70% by weight, still more preferably 85 to 75% by weight. The amount of the mixed solvent to be used is preferably 100 to 350 parts by weight, more preferably 150 to 250 parts by weight, relative to 100 parts by weight of compound A. When compound A is added to the mixed solvent (5) composed of the linear

ketone solvent and water and then the resultant solution is warmed, a solution having compound A dissolved in the mixed solvent (5) composed of the linear ketone solvent and water can be prepared. However, when the prepared solution is cooled without any other procedure, crystals may not be sometimes precipitated depending on the amount of the solvent or the amount of water, or the yield of the crystals may be sometimes poor. For these reasons, it is preferred to cause crystals to be precipitated while adding water to a solution prepared by dissolving compound A in a linear ketone solvent having 3 to 8 carbon atoms in total, or to cause crystals to be precipitated by cooling the solution after the addition of water. The ratio of the weight of water to the weight of the linear ketone solvent in a crystallization liquid after the addition of water is such that the amount of water is preferably 150 to 400 parts by weight, more preferably 230 to 300 parts by weight, relative to 100 parts by weight of the linear ketone solvent. In the case where water is added to the mixed solvent, the crystal precipitation temperature to be employed is preferably 55 to 65°C, more preferably about 60°C. The period of time to be employed for the addition of water is preferably 1 to 5 hours, more preferably 1.5 to 2.5 hours, still more preferably about 2 hours.

In the crystallization procedure using any one of the solvents (1) to (5), the cooling rate to be employed during the crystallization or after the precipitation of crystals is preferably 5 to 15°C, more preferably 7 to 12°C, per 1 hour. In the precipitation of crystals, seed crystals may not be used. However, it is preferred to use seed crystals. In this case, the seed crystals to be used may be crystals that have been precipitated without seed crystals. The final cooling temperature is preferably 20 to 60°C, more preferably 25 to 35°C. After cooling to this temperature, the precipitated crystals are separated by a filtration procedure.

<Drying step>

The solvent used in the crystallization can be removed by drying the crystals produced by the crystallization. In the drying of the crystals obtained by crystallization, the drying procedure may be carried out under ambient pressure or under reduced pressure. In the case where it is intended to carry out the drying procedure on an industrial scale, it is preferred to carry out the drying procedure under reduced pressure, because the solvent used in the crystallization can be removed more effectively. It is preferred to carry out the drying procedure at 60 to 120°C under reduced pressure, more preferably at 70 to 110°C under reduced pressure.

<Crystal of the present invention>

The crystal of the present invention is characterized by having an endothermic peak top temperature within the range of 215 to 220°C as measured by differential scanning calorimetry and also having an aerated bulk density within the range of 0.3 to 0.6 g/$cm^3$.

The term "aerated bulk density" as used herein generally refers to a value which is measured by introducing granules of interest into a container having a specific volume while avoiding the formation of void spaces in the container and while avoiding the application of an external force such as vibrations to the container, then measuring the weight of the granules and then dividing the weight by the volume of the container. For example, the aerated bulk density can be calculated from the results measured by the below-mentioned method or the like using a measuring instrument for multi functional powder properties (e.g., model "Multi Tester MT-1001", manufactured by SEISHIN ENTERPRISE Co., Ltd.) or the like. It is preferred that the aerated bulk density in the crystal of the present invention is a larger value within the range from 0.3 to 0.6 g/$cm^3$. The lower limit in this numerical value range is preferably 0.33 g/$cm^3$ or more, more preferably 0.4 g/$cm^3$ or more. The upper limit in this numerical value range is preferably a value closer to 0.6 g/$cm^3$, and may be a value about 0.55 g/$cm^3$ or less or a value about 0.5 g/$cm^3$ or less.

In the crystals produced in Comparative Examples mentioned below, even though the endothermic peak top temperatures as determined by differential scanning calorimetry were respectively 210°C, 214°C and 215°C, the aerated bulk densities fell within the range from 0.12 g/$cm^3$ to 0.23 g/$cm^3$. Therefore, it is demonstrated that the crystals of the present invention are greatly improved in aerated bulk density compared with the crystals of Comparative Examples. Namely, the crystal of the present invention does not undergo the formation of dusts, cannot be adhered to a production facility or does not cause clogging in the production facility. In addition, the crystal enables the reduction in the volume of a reaction vessel to be used when the crystal is used as a reaction raw material or the reduction in the volume of a container to be used for transportation of the crystal. Therefore, the crystal can exert excellent effects with respect to operability, such as the improvement in productivity and the reduction in transportation cost.

The crystal of the present invention has reduced coloring which is an excellent characteristic property. More specifically, it is preferred that the Hazen color number (APHA) of a 30% by weight solution of the crystal, which is prepared by dissolving the crystal in tetrahydrofuran (purity: 97% or more), is 100 or less. Particularly, the Hazen color number (APHA) is more preferably 80 or less, still more preferably 60 or less, most preferably 30 or less. In order to produce the crystal of the present invention which has reduced coloring, the above crystallization step is preferably carried out under an inert gas atmosphere such as a nitrogen atmosphere, and the above drying step is preferably

carried out under an inert gas atmosphere such as a nitrogen atmosphere or under reduced pressure.

## EXAMPLES

Hereinafter, the present invention will be described in more detail. However, the present invention is not intended to be limited to these examples.

The analysis methods are as follows.

<Analysis methods>

### 1. Differential scanning calorimetry (DSC)

Crystals were weighed accurately in an aluminum pan, and were then measured using aluminum oxide as a control under the following manipulation conditions using a differential scanning calorimeter (manufactured by Shimadzu Corporation; DSC-60).

(Manipulation conditions)

Temperature rising rate: 10°C/min
Measurement temperature range: 30 to 260°C
Measurement atmosphere: open, nitrogen 50 mL/min
Sample amount: 3 mg ± 1 mg

### 2. Aerated bulk density

The aerated bulk density was determined using a measuring instrument for multi functional powder properties (e.g., model "Multi Tester MT-1001", manufactured by SEISHIN ENTERPRISE Co., Ltd.) as follows: crystals were gently introduced into a measurement cell having a volume of 20 $cm^3$ through a sieve while avoiding the formation of air voids, then the weight a (g) of the crystals in the cell at a time point when the measurement cell was filled with the crystals was measured, and the aerated bulk density was calculated in accordance with the following calculation formula.

[Calculation Formula]

$$\text{Aerated bulk density (g/cm}^3\text{)} = \text{(weight of crystals a (g))} \div 20\ \text{cm}^3$$

### 3. Powder X-ray diffraction (XRD)

Crystals (0.1 g) were packed in a sample packing section in a glass test plate, and the powder X-ray diffraction of the crystals was measured under the following conditions using a powder X-ray diffraction measurement device (manufactured by Rigaku Corporation; SmartLab).

X-ray source: CuKα
Scan axis: 2θ/θ
Mode: continuous
Measurement range: 2θ = 5° to 70°
Step: 0.01°
Speed measurement time: 2θ = 2°/min
IS :1/2
RS :20.00mm
Output: 40 kV-30 mA

### 4. Hue (APHA)

Crystals were dissolved in tetrahydrofuran (manufactured by FUJIFILM Wako Pure Chemical Corporation, purity: 97% or more) to prepare a 30% by weight solution of the crystals, then the "standard calibration" for the below-mentioned measurement device was carried out with tetrahydrofuran, and then the dissolution color of the 30% by weight solution was measured.

Measurement device: TZ 6000 manufactured by Nippon Denshoku Industries, Co., Ltd.

<Example 1>

Crystals of 2,2'-bis(carboxymethoxy)-1,1'-binaphthyl (compound A) (Ver. 1)

1,1'-Binaphthalene-2,2'-diol (referred to as "compound b", hereinafter) (52 g), N-methylpyrrolidone (156 g), potassium carbonate (58 g) and potassium iodide (5.2 g) were charged in a four-necked flask, and the resultant mixture was heated to 70°C and was then stirred at the same temperature for 1 hour. Ethyl chloroacetate (62 g) was added dropwise to the reaction solution while keeping the temperature of the reaction solution at 65 to 70°C. The reaction solution was stirred for 4 hours, then water (156 g) and potassium hydroxide (30 g) were added to the solution, and then the resultant solution was stirred at 85 to 90°C for 13 hours. Subsequently, water (130 g) and methyl isobutyl ketone (referred to as "MIBK", hereinafter) (156 g) were added to the solution, then the resultant solution was stirred for 15 minutes while keeping the temperature of the solution at 80 to 85°C, and then an aqueous layer was removed and was transferred to another four-necked reaction flask. MIBK (416 g) and water (416 g) were added to the aqueous layer, then concentrated hydrochloric acid (100 g) was added dropwise to the solution while keeping the temperature of the solution at 80 to 85°C, and the resultant solution was stirred at the same temperature for 30 minutes. Subsequently, the solution was allowed to stand, then an aqueous layer was removed from the solution to obtain an oily layer, then water was added to the oily layer, then the resultant solution was stirred, and then the solution was allowed to stand to remove an aqueous layer to obtain an oily layer. Water and MIBK (252 g) were distilled away from the oily layer by distillation over 4 hours under ambient pressure while stirring. One hour after the start of the distillation, crystals were precipitated without the need to add seed crystals. Subsequently, the solution was cooled to 25°C at a cooling rate of 10°C per hour to cause the precipitation of crystals, then the crystals were filtrated off and were then dried to produce powdery crystals of compound A (60.7 g) (yield: 82%).

With respect to the powdery crystals, the purity determined by high-performance liquid chromatography measurement was 98.7%, the endothermic peak top temperature measured by differential scanning calorimetry was 217°C, the aerated bulk density was 0.34 g/$cm^3$, and the hue (APHA) was 50.

The chart diagram showing the data of the differential scanning calorimetry is shown in Fig. 1 (amount of sample: 2.311 mg).

<Example 2>

Crystals of compound A (Ver. 2)

Compound b (1213 g), acetonitrile (3638 g), potassium carbonate (1346 g) and potassium iodide (121 g) were charged in a four-necked flask, and the resultant mixture was heated to 70°C and was then stirred at the same temperature for 1 hour. A mixed solution of ethyl chloroacetate (1460 g) and N-methylpyrrolidone (13 g) was prepared, and then the mixed solution was added dropwise to the reaction solution while keeping the temperature of the reaction solution at 70 to 80°C. The resultant solution was stirred for 6 hours, then water (3032 g) was added to the solution, then the resultant solution was heated to 70°C, and then an aqueous layer was removed therefrom. Subsequently, a 35% aqueous potassium hydroxide solution (3392 g) was added dropwise to the reaction solution while keeping the temperature of the reaction solution at 70 to 80°C. Two hours after, the reaction solution was cooled gradually, and then the reaction solution was filtrated at 25°C to obtain crystals of a potassium salt of compound A (2180 g).

Subsequently, the crystals were subjected to a crystallization step under a nitrogen atmosphere. A portion (2051 g) (amount of solvent attached: about 16% by weight) of the crystals of the potassium salt was used, and water (3430 g) and MIBK (9702 g) were charged in a four-necked flask, and then the resultant solution was heated to 80°C to dissolved the components. Concentrated hydrochloric acid (1207 g) was added dropwise to the reaction solution while keeping the temperature of the reaction solution at 80 to 85°C, and the resultant solution was stirred at the same temperature for 30 minutes. Subsequently, an aqueous layer was removed, and then water was added to the residue to wash the residue with water to obtain an oily layer. Subsequently, water and MIBK (4713 g) were distilled away from the oily layer under ambient pressure by distillation over 8 hours. At a point of time where two hours was elapsed after the start of the distillation, the crystals (1 g) obtained in Example 1 were added as seed crystals to the resultant solution to carry out crystallization. The crystallization liquid was cooled to 25°C at a cooling rate of 10°C per hour and was filtrated, and then the residue was dried under reduced pressure to obtain crystals of compound A (1392 g) (yield: 86.8%).

With respect to the crystals, the purity determined by high-performance liquid chromatography measurement was 99.9%, the endothermic peak top temperature measured by differential scanning calorimetry was 217°C, the aerated

bulk density was 0.45 g/$cm^3$, and the hue (APHA) was 30. The characteristic 2θ (deg) values in XRD were as follows: 8.1, 9.2, 14.8, 16.2, 17.5, 18.2, 18.5, 22.7, 23.4, 24.4, 26.9, 27.5, 31.5, 36.3 and 39.2.

The chart diagram showing the data of the differential scanning calorimetry is shown in Fig. 2 (amount of sample: 2.952 mg).

<Example 3>

The step of crystallization was carried out using compound A obtained in Example 2 under a nitrogen atmosphere. Compound A (607 g) and an 80% aqueous acetone solution (1220 g) were charged in a four-necked flask, and then the resultant solution was heated to 50°C to dissolve compound A in the aqueous solution. Water (1100 g) was added to the solution while keeping the internal temperature at 55 to 60°C. Subsequently, the crystals (1 g) obtained in Example 1 were added as seed crystals to the solution, and then water (1100 g) was added to the resultant solution over 2 hours while keeping the temperature of the solution at 55 to 60°C to perform crystallization. The crystallization liquid was cooled to 25°C at a cooling rate of 10°C per hour and was filtrated, and then the residue was dried under reduced pressure to obtain crystals of the present invention (552 g) (purification yield: 90.9%).

With respect to the crystals, the purity determined by high-performance liquid chromatography measurement was 99.9%, the endothermic peak top temperature measured by differential scanning calorimetry was 218°C, the aerated bulk density was 0.46 g/$cm^3$, and the hue (APHA) was 20. The characteristic 2θ (deg) values in XRD were as follows: 8.1, 9.2, 14.8, 16.2, 18.5, 23.4, 24.4, 26.9, 27.5, 31.5, 36.3 and 39.2.

The chart diagram showing the data of the differential scanning calorimetry is shown in Fig. 3 (amount of sample: 1.988 mg).

<Example 4>

The step of crystallization was carried out using compound A obtained in Example 2 under a nitrogen atmosphere. Compound A (20 g) and 2-octanone (120 g) were charged in a four-necked flask, the resultant solution was heated to 145°C to dissolve compound A, and the resultant solution was cooled at a cooling rate of 10°C per hour. The precipitation of crystals was observed at 127°C. Subsequently, the crystallization liquid was cooled to 25°C and was filtrated, and then the residue was dried under reduced pressure to obtain crystals of the present invention (18.5 g) (purification yield: 92.5%).

With respect to the crystals, the purity determined by high-performance liquid chromatography measurement was 99.9%, the endothermic peak top temperature measured by differential scanning calorimetry was 215°C, the aerated bulk density was 0.35 g/$cm^3$, and the hue (APHA) was 20. The characteristic 2θ (deg) values in XRD were as follows: 8.1, 9.2, 14.8, 16.1, 18.2, 22.5, 23.4, 24.3, 26.8 and 36.3.

<Example 5>

The step of crystallization was carried out using compound A obtained in Example 2 under a nitrogen atmosphere. Compound A (20 g) and a 90% aqueous methyl ethyl ketone solution (38.7 g) were charged in a four-necked flask, the resultant solution was heated to 72°C to dissolve compound A, and the resultant solution was cooled at a cooling rate of 10°C per hour. The precipitation of crystals was observed at 55°C. Subsequently, the crystallization liquid was cooled to 25°C and was filtrated, and then the residue was dried under reduced pressure to obtain crystals of the present invention (6.5 g).

With respect to the crystals, the purity determined by high-performance liquid chromatography measurement was 99.9%, the endothermic peak top temperature measured by differential scanning calorimetry was 216°C, the aerated bulk density was 0.32 g/$cm^3$, and the hue (APHA) was 10. The characteristic 2θ (deg) values in XRD were as follows: 9.2, 14.6, 16.2, 18.2, 22.5, 23.3, 24.3, 26.8 and 36.3.

<Example 6>

The step of crystallization was carried out using compound A obtained in Example 2 under a nitrogen atmosphere. Compound A (20.1 g) and cyclohexanone (20.2 g) were charged in a four-necked flask, the resultant solution was heated to 146°C to dissolve compound A, and the resultant solution was cooled at a cooling rate of 10°C per hour. The precipitation of crystals was observed at 103°C. Subsequently, the crystallization liquid was cooled to 25°C and was filtrated, and then the residue was dried under reduced pressure to obtain crystals of the present invention (13.7 g).

With respect to the crystals, the purity determined by high-performance liquid chromatography measurement was 99.9%, the endothermic peak top temperature measured by differential scanning calorimetry was 215°C, the aerated bulk density was 0.34 g/$cm^3$, and the hue (APHA) was 40. The characteristic 2θ (deg) values in XRD were as follows:

9.2, 14.8, 16.2, 18.2, 23.4, 24.3, 26.8 and 36.3.

<Example 7>

The step of crystallization was carried out using compound A obtained in Example 2 under a nitrogen atmosphere. Compound A (15 g) and tetrahydrofuran (15.9 g) were charged in a four-necked flask, the resultant solution was heated to 65°C to dissolve compound A, and the resultant solution was cooled at a cooling rate of 10°C per hour. The precipitation of crystals was observed at 59°C. Subsequently, the crystallization liquid was cooled to 25°C and was filtrated, and then the residue was dried under reduced pressure to obtain crystals of the present invention (7.5 g).
With respect to the crystals, the purity determined by high-performance liquid chromatography measurement was 99.8%, the endothermic peak top temperature measured by differential scanning calorimetry was 215°C, the aerated bulk density was 0.43 g/cm$^3$, and the hue (APHA) was 60. The characteristic 2$\theta$ (deg) values in XRD were as follows: 9.2, 14.8, 16.2, 18.2, 18.4, 22.5, 23.4, 24.3, 26.8 and 36.3.

<Example 8>

The step of crystallization was carried out using compound A obtained in Example 2 under a nitrogen atmosphere. Compound A (15.1 g) and 1,4-dioxane (17.5 g) were charged in a four-necked flask, the resultant solution was heated to 101°C to dissolve compound A, and the resultant solution was cooled at a cooling rate of 10°C per hour. The precipitation of crystals was observed at 70°C. Subsequently, the crystallization liquid was cooled to 25°C and was filtrated, and then the residue was dried under reduced pressure to obtain crystals of the present invention (8.0 g).
With respect to the crystals, the purity determined by high-performance liquid chromatography measurement was 99.4%, the endothermic peak top temperature measured by differential scanning calorimetry was 215°C, the aerated bulk density was 0.41 g/cm$^3$, and the hue (APHA) was 80. The characteristic 2$\theta$ (deg) values in XRD were as follows: 8.1, 14.6, 16.1, 18.2, 22.5, 23.4, 24.4, 26.8 and 36.3.

<Example 9>

The step of crystallization was carried out using compound A obtained in Example 2 under a nitrogen atmosphere. Compound A (15 g) and $\gamma$-butyrolactone (10 g) were charged in a four-necked flask, the resultant solution was heated to 140°C to dissolve compound A, and the resultant solution was cooled at a cooling rate of 10°C per hour. The precipitation of crystals was observed at 103°C. Subsequently, the crystallization liquid was cooled to 25°C and was filtrated, and then the residue was dried under reduced pressure to obtain crystals of the present invention (13.1 g).
With respect to the crystals, the purity determined by high-performance liquid chromatography measurement was 99.8%, the endothermic peak top temperature measured by differential scanning calorimetry was 215°C, the aerated bulk density was 0.33 g/cm$^3$, and the hue (APHA) was 130. The characteristic 2$\theta$ (deg) values in XRD were as follows: 9.2, 14.7, 16.1, 18.2, 22.5, 23.3, 24.3, 26.8 and 36.3.

<Example 10>

The step of crystallization was carried out using compound A obtained in Example 2 under a nitrogen atmosphere. Compound A (20 g) and $\gamma$-valerolactone (20 g) were charged in a four-necked flask, the resultant solution was heated to 135°C to dissolve compound A, and the resultant solution was cooled at a cooling rate of 10°C per hour. The precipitation of crystals was observed at 123°C. Subsequently, the crystallization liquid was cooled to 25°C and was filtrated, and then the residue was dried under reduced pressure to obtain crystals of the present invention (16.7 g).
With respect to the crystals, the purity determined by high-performance liquid chromatography measurement was 99.7%, the endothermic peak top temperature measured by differential scanning calorimetry was 215°C, and the aerated bulk density was 0.44 g/cm$^3$. The characteristic 2$\theta$ (deg) values in XRD were as follows: 8.0, 14.6, 16.1, 18.1, 23.3, 24.3, 26.9 and 36.2.

<Comparative Example 1>

Compound b (4.0 g), potassium carbonate (34.8 g), bromoacetic acid (20.1 g) and methanol (118 mL) were charged in a four-necked flask, and the resultant solution was allowed to react under reflux for 6 hours. Methanol was removed from the reaction solution, then water was added to the resultant solution, and then a 3-N aqueous hydrochloric acid solution was added to the resultant solution until the solution had a pH value of 1. The reaction solution was transferred to a separatory funnel and was then extracted with a benzene/(diethyl ether) mixture (1 : 1) (30 mL) five times. Extracted organic layers were combined, the resultant solution was dehydrated with anhydrous sodium sulfate,

and the solvent was distilled away from the dehydrated solution with an evaporator to obtain a solid matter. The above-mentioned reactions were repeated until a solid matter in an amount required for the analyses was obtained.

With respect to the solid matter, the purity determined by high-performance liquid chromatography measurement was 96.0%, the endothermic peak top temperature measured by differential scanning calorimetry was 214°C, and the aerated bulk density was 0.12 g/cm$^3$.

The chart diagram showing the data of the differential scanning calorimetry is shown in Fig. 4 (amount of sample: 2.131 mg).

<Comparative Example 2>

Compound b (4.0 g), potassium carbonate (34.8 g), bromoacetic acid (20.1 g) and methanol (118 mL) were charged in a four-necked flask, and the resultant solution was allowed to react under reflux for 6 hours. Methanol was removed from the reaction solution, then water was added to the resultant solution, and then a 3-N aqueous hydrochloric acid solution was added to the resultant solution until the solution had a pH value of 1. A benzene/(petroleum ether) mixed solution (1:1) (150 mL) was added to a precipitated solid matter. However, the solid matter was not dissolved in the mixed solution. Therefore, the solution was filtrated without any other procedure to obtain a solid matter. The above-mentioned reactions were repeated until a solid matter in an amount required for the analyses was obtained.

With respect to the solid matter, the purity determined by high-performance liquid chromatography measurement was 98.3%, the endothermic peak top temperature measured by differential scanning calorimetry was 210°C, and the aerated bulk density was 0.14 g/cm$^3$.

The chart diagram showing the data of the differential scanning calorimetry is shown in Fig. 5 (amount of sample: 2.536 mg).

<Comparative Example 3>

The step of crystallization was carried out using compound A obtained in Example 2 under a nitrogen atmosphere. Compound A (20 g) and methyl ethyl ketone (158.3 g) were charged in a four-necked flask, the resultant solution was heated to 79°C to dissolve compound A, and the resultant solution was cooled at a cooling rate of 10°C per hour. The precipitation of crystals was observed at 44°C. Subsequently, the crystallization liquid was cooled to 25°C and was filtrated, and then the residue was dried under reduced pressure to obtain crystals of the present invention (11.2 g).

With respect to the crystals, the purity determined by high-performance liquid chromatography measurement was 99.9%, the endothermic peak top temperature measured by differential scanning calorimetry was 215°C, the aerated bulk density was 0.23 g/cm$^3$, and the hue (APHA) was 30. The characteristic 2θ (deg) values in XRD were as follows: 9.2, 14.8, 16.3, 18.2, 22.5, 23.4, 24.4, 26.8 and 36.3.

<Comparative Example 4>

The step of crystallization was carried out using compound A obtained in Example 2 under a nitrogen atmosphere. Compound A (15 g) and butyl acetate (237.1 g) were charged in a four-necked flask, the resultant solution was heated to 125°C to dissolve compound A, and the resultant solution was cooled at a cooling rate of 10°C per hour. The precipitation of crystals was observed at 110°C. Subsequently, the crystallization liquid was cooled to 25°C and was filtrated, and then the residue was dried under reduced pressure to obtain crystals of the present invention (13.0 g).

With respect to the crystals, the purity determined by high-performance liquid chromatography measurement was 99.9%, the endothermic peak top temperature measured by differential scanning calorimetry was 215°C, the aerated bulk density was 0.18 g/cm$^3$, and the hue (APHA) was 30. The characteristic 2θ (deg) values in XRD were as follows: 9.2, 14.8, 16.1, 18.2, 22.5, 23.3, 24.3, 26.8 and 36.3.

<Comparative Example 5>

The step of crystallization was carried out using compound A obtained in Example 2 under a nitrogen atmosphere. Compound A (10 g) and cyclopentyl methyl ether (210.1 g) were charged in a four-necked flask, the resultant solution was heated to 103°C to dissolve compound A, and the resultant solution was cooled at a cooling rate of 10°C per hour. The precipitation of crystals was observed at 60°C. Subsequently, the crystallization liquid was cooled to 25°C and was filtrated, and then the residue was dried under reduced pressure to obtain crystals of the present invention (6.5 g).

With respect to the crystals, the purity determined by high-performance liquid chromatography measurement was 99.2%, the endothermic peak top temperature measured by differential scanning calorimetry was 215°C, the aerated bulk density was 0.23 g/cm$^3$, and the hue (APHA) was 90. The characteristic 2θ (deg) values in XRD were as follows: 9.2, 14.7, 16.2, 18.2, 22.5, 23.4, 24.3, 26.8 and 36.3.

## Claims

1. A crystal of 2,2'-bis(carboxymethoxy)-1,1'-binaphthyl, the crystal having an endothermic peak top temperature within a range of 215 to 220°C as measured by differential scanning calorimetry and also having an aerated bulk density within a range of 0.3 to 0.6 g/$cm^3$.

2. The crystal of 2,2'-bis(carboxymethoxy)-1,1'-binaphthyl according to claim 1, wherein Hazen color number (APHA) of a 30% by weight solution prepared by dissolving the crystal in tetrahydrofuran is 100 or less.

3. A method for producing the crystal according to claim 1 or 2, the method comprising carrying out crystallization with any one solvent selected from following solvents (1) to (5):

   (1) at least one solvent selected from linear ketone solvents each having 5 to 8 carbon atoms in total;
   (2) at least one solvent selected from cyclic ketone solvents each having 5 to 8 carbon atoms in total;
   (3) at least one solvent selected from cyclic ether solvents each having 4 to 8 carbon atoms in total;
   (4) at least one solvent selected from cyclic ester solvents each having 4 to 8 carbon atoms in total; and
   (5) a mixed solvent of at least one solvent selected from linear ketone solvents each having 3 to 8 carbon atoms in total and water.

4. The method for producing according to claim 3, wherein the method comprises carrying out crystallization with any one solvent selected from following solvents (1') to (5'):

   (1') methyl isobutyl ketone or 2-octanone;
   (2') cyclohexanone;
   (3') tetrahydrofuran or 1,4-dioxane;
   (4') γ-butyrolactone or γ-valerolactone; and
   (5') a mixed solvent of acetone and water or a mixed solvent of methyl ethyl ketone and water.

## Patentansprüche

1. Kristall aus 2,2'-Bis(carboxymethoxy)-1,1'-binaphthyl, wobei der Kristall eine endotherme Peak-Toptemperatur in einem Bereich von 215 bis 220 °C, gemessen durch Differential-Scanning-Kalorimetrie, und auch eine mit Luft durchsetzte Schüttdichte im Bereich von 0,3 bis 0,6 g/$cm^3$ besitzt.

2. Der Kristall aus 2,2'-Bis(carboxymethoxy)-1,1'-binaphthyl nach Anspruch 1, wobei die Hazen-Farbzahl (APHA) einer 30 gewichtsprozentigen Lösung, die durch Lösen des Kristalls in Tetrahydrofuran hergestellt wurde, 100 oder weniger ist.

3. Verfahren zum Herstellen des Kristalls nach Anspruch 1 oder 2, wobei das Verfahren das Durchführen der Kristallisation mit irgendeinem Lösungsmittel, ausgewählt aus den folgenden Lösungsmitteln (1) bis (5), umfasst:

   (1) mindestens ein Lösungsmittel, ausgewählt aus linearen Ketonlösungsmitteln, jedes besitzend insgesamt 5 bis 8 Kohlenstoffatome;
   (2) mindestens ein Lösungsmittel, ausgewählt aus cyclischen Ketonlösungsmitteln, jedes besitzend insgesamt 5 bis 8 Kohlenstoffatome;
   (3) mindestens ein Lösungsmittel, ausgewählt aus cyclischen Etherlösungsmitteln, jedes besitzend insgesamt 4 bis 8 Kohlenstoffatome;
   (4) mindestens ein Lösungsmittel, ausgewählt aus cyclischen Esterlösungsmitteln, jedes besitzend insgesamt 4 bis 8 Kohlenstoffatome; und
   (5) ein gemischtes Lösungsmittel aus mindestens einem Lösungsmittel, ausgewählt aus linearen Ketonlösungsmitteln, jedes besitzend insgesamt 3 bis 8 Kohlenstoffatome, und Wasser.

4. Das Verfahren zur Herstellung nach Anspruch 3, wobei das Verfahren das Durchführen der Kristallisation mit irgendeinem Lösungsmittel, ausgewählt aus den folgenden Lösungsmitteln (1') bis (5'), umfasst:

   (1') Methylisobutylketon oder 2-Octanon;
   (2') Cyclohexanon;

(3') Tetrahydrofuran oder 1,4-Dioxan;
(4') γ-Butyrolacton oder γ-Valerolacton; und
(5') ein gemischtes Lösungsmittel aus Aceton und Wasser oder ein gemischtes Lösungsmittel aus Methylethylketon und Wasser.

## Revendications

1. Cristal de 2,2'-bis(carboxyméthoxy)-1,1'-binaphtyle, le cristal ayant une température au sommet du pic endothermique dans une plage de 215 à 220 °C ainsi que mesurée par analyse calorimétrique à compensation de puissance et ayant également une masse volumique apparente à l'état aéré dans une plage de 0,3 à 0,6 g/$cm^3$.

2. Cristal de 2,2'-bis(carboxyméthoxy)-1,1'-binaphtyle selon la revendication 1, l'indice de couleur Hazen (APHA) d'une solution à 30 % en poids préparée en dissolvant le cristal dans du tétrahydrofurane étant inférieur ou égal à 100.

3. Procédé de production du cristal selon la revendication 1 ou 2, le procédé comprenant la réalisation d'une cristallisation avec un solvant quelconque choisi parmi les solvants (1) à (5) suivants :

(1) au moins un solvant choisi parmi des solvants cétoniques linéaires ayant chacun 5 à 8 atomes de carbone au total ;
(2) au moins un solvant choisi parmi des solvants cétoniques cycliques ayant chacun 5 à 8 atomes de carbone au total ;
(3) au moins un solvant choisi parmi des solvants éther cycliques ayant chacun 4 à 8 atomes de carbone au total ;
(4) au moins un solvant choisi parmi des solvants ester cycliques ayant chacun 4 à 8 atomes de carbone au total ; et
(5) un solvant mixte constitué d'eau et d'au moins un solvant choisi parmi des solvants cétoniques linéaires ayant chacun 3 à 8 atomes de carbone au total.

4. Procédé de production selon la revendication 3, le procédé comprenant la réalisation d'une cristallisation avec un solvant quelconque choisi parmi les solvants (1') à (5') suivants :

(1') méthylisobutylcétone ou 2-octanone ;
(2') cyclohexanone ;
(3') tétrahydrofurane ou 1,4-dioxane ;
(4') γ-butyrolactone ou γ-valérolactone ; et
(5') un solvant mixte constitué d'acétone et d'eau ou un solvant mixte constitué de méthyléthylcétone et d'eau.

[Fig. 1]

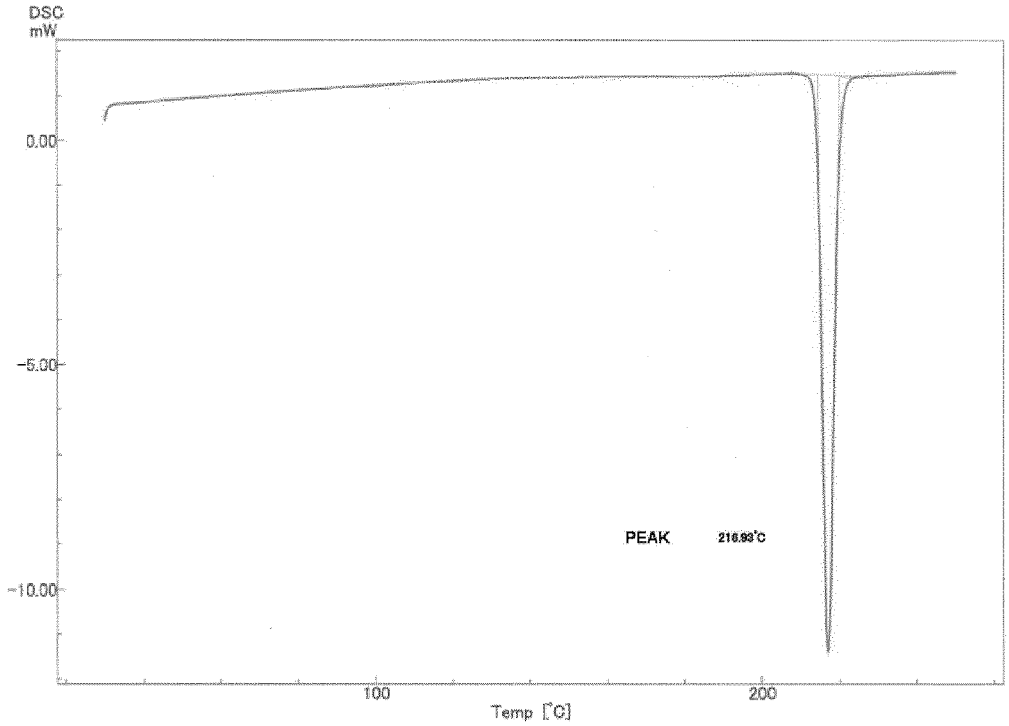
DSC
mW
0.00
-5.00
-10.00
100
200
Temp [°C]
PEAK
216.93°C

[Fig. 2]

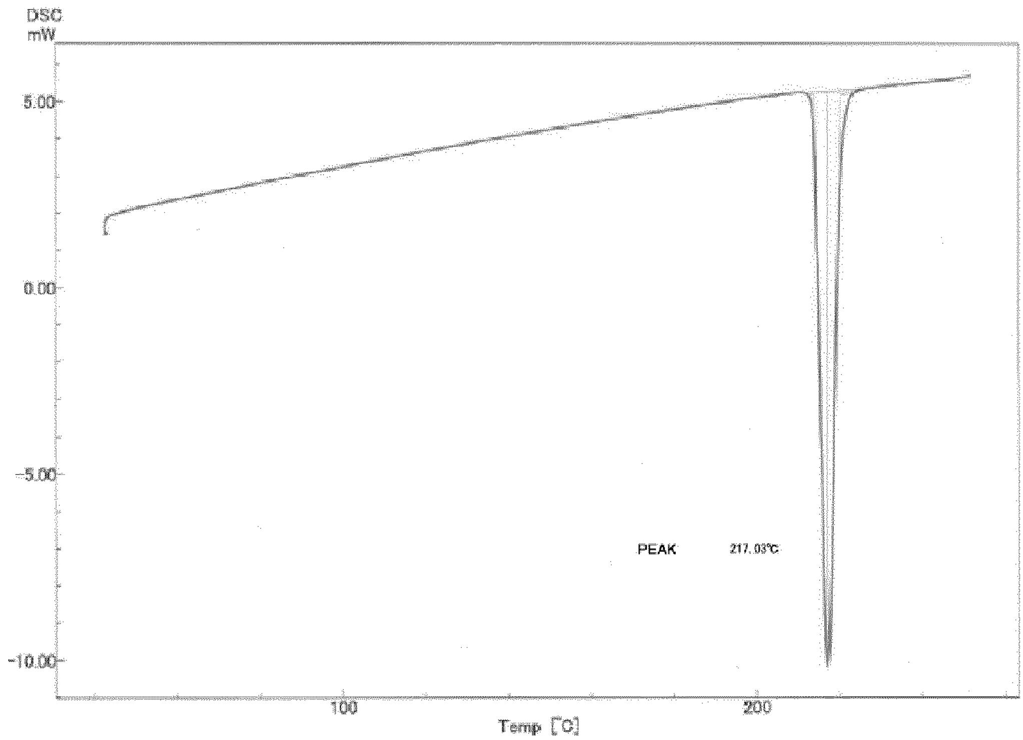
DSC
mW
5.00
0.00
-5.00
-10.00
100
200
Temp [°C]
PEAK
217.03°C

[Fig. 3]

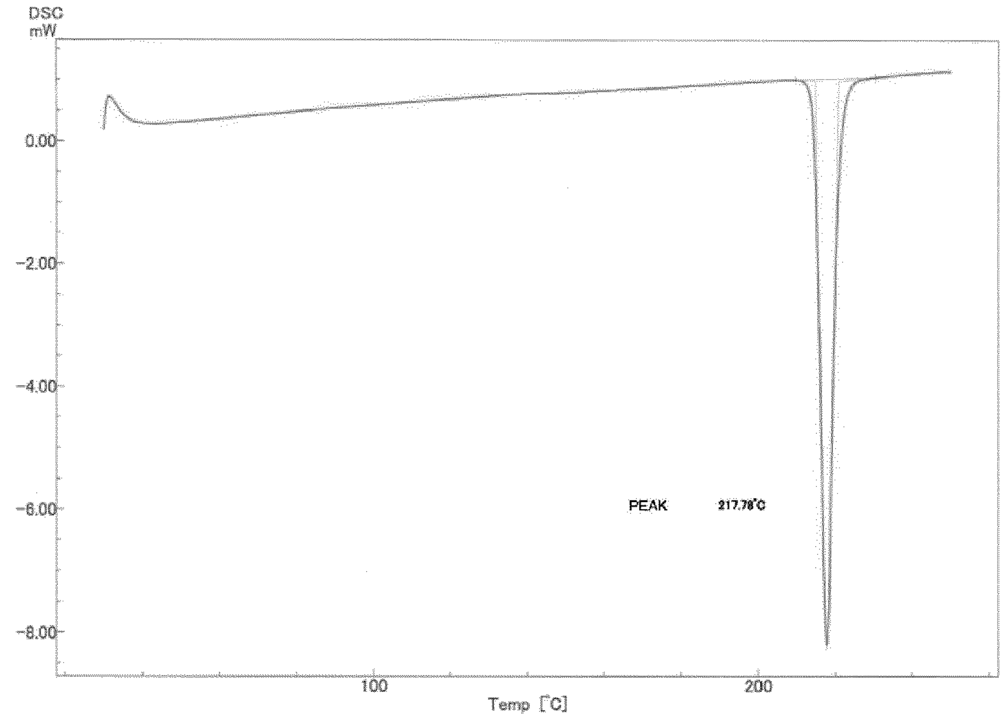
DSC
mW
0.00
-2.00
-4.00
-6.00
-8.00
PEAK 217.78°C
100
200
Temp [°C]

[Fig. 4]

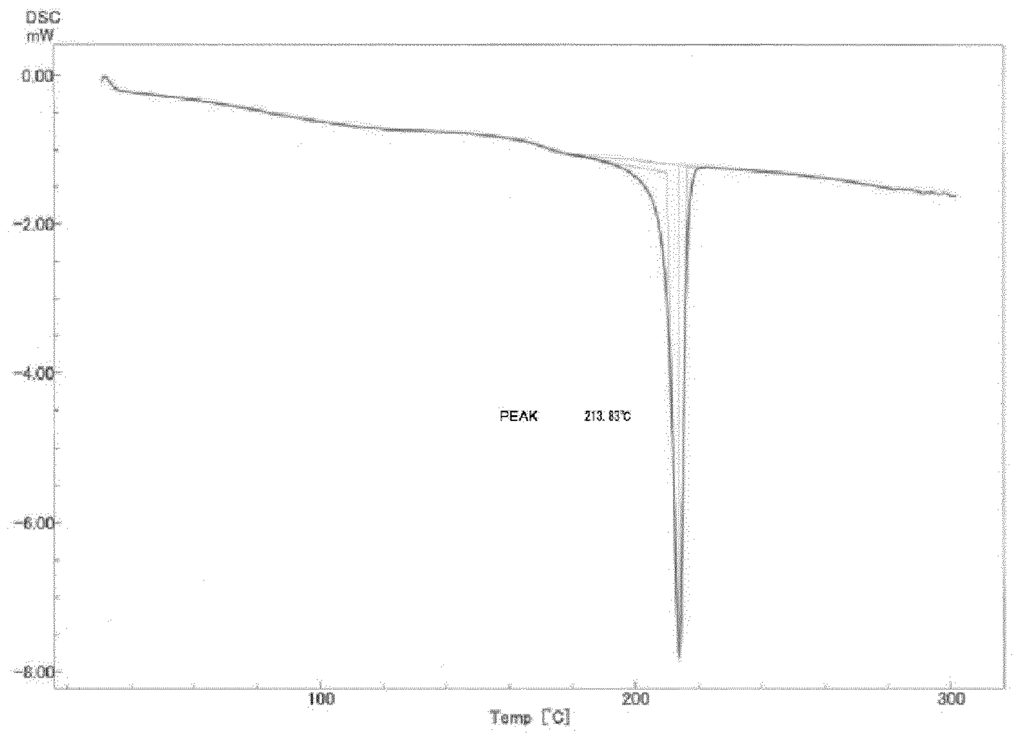
DSC
mW
0.00
-2.00
-4.00
-6.00
-8.00
PEAK 213. 83°C
100
200
300
Temp [°C]

[Fig. 5]

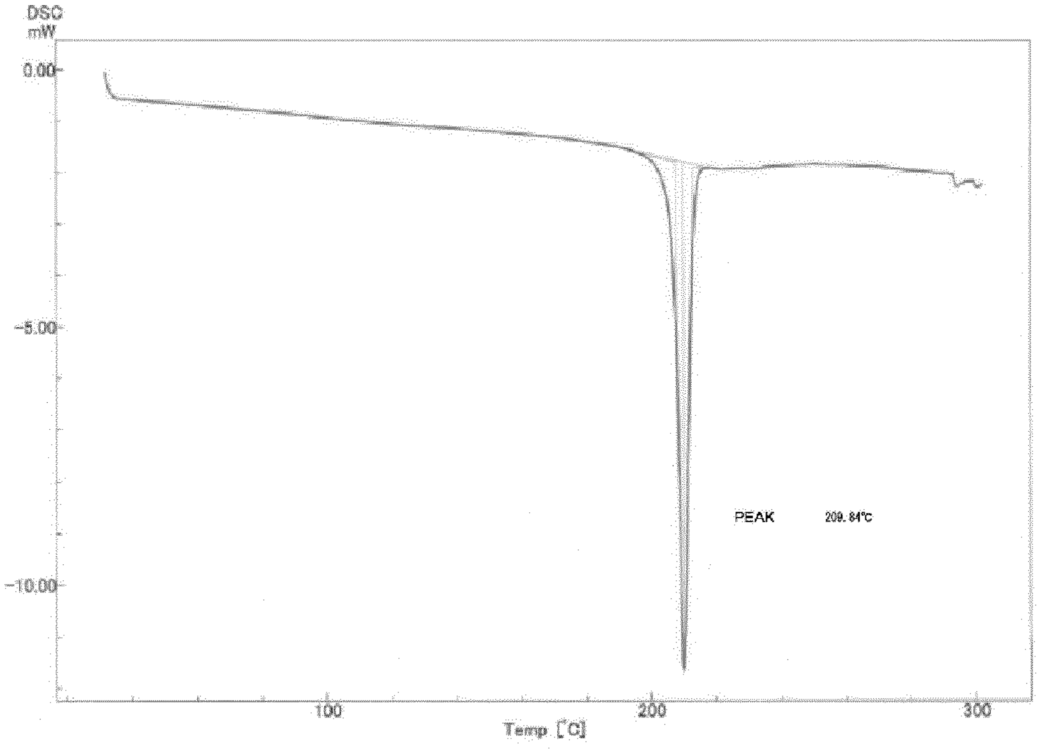

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001072872 A
- JP 2018002893 A
- JP 2018002894 A
- JP 2018002895 A
- JP 2008024650 A


### Non-patent literature cited in the description


- *The Journal of Physical Chemistry B,* 2011, vol. 115 (26), 8597-8608